# EUROPEAN PATENT SPECIFICATION

(11) **EP 1 874 728 B1**
(45) Date of publication and mention of the grant of the patent: **19.03.2014**
(21) Application number: 06746046.9
(22) Date of filing: 27.04.2006
(51) Int. Cl.: C07D 207/267, C07D 207/26

(54) **COMPOSITION OF N-ALKENYL CARBOXYLIC ACID TERTIARY AMIDE**
ZUSAMMENSETZUNG MIT TERTIÄREM N-ALKENYL-CARBOXYLSÄUREAMID
PREPARATION CONTENANT UN N-ALCENYLAMIDE TERTIAIRE D'ACIDE CARBOXYLIQUE

(30) Priority: 28.04.2005 JP 2005130641
(43) Date of publication of application: 09.01.2008
(73) Proprietor: Nippon Shokubai Co.,Ltd., Osaka-shi, Osaka 541-0043 (JP)
(72) Inventor: UGAMURA, Shukichi, 5640035 (JP); TAKEMATSU, Kenichi, 1320024 (JP)
(74) Representative: Mai, Dörr, Besier
(86) International application number: PCT/JP2006/309214
(87) International publication number: WO 2006/118330

(56) References cited:
- EP-A- 0 467 851
- EP-A- 1 518 916
- EP-A1- 1 950 230
- WO-A-00/37412
- WO-A-99/55797
- WO-A-02/057372
- DE-A1- 10 346 135
- US-A- 3 959 358
- US-A- 5 258 138
- US-A- 5 340 488
- US-A- 5 461 159
- US-A1- 2002 091 229
- US-A1- 2005 079 228
- US-B1- 6 649 717
- US-B1- 6 911 216

## Description

### Technical Field:

The present invention relates to a composition of N-vinyl-pyrrolidone (NVP), having stability for a long period, by the addition of specified amines.

### Background Art:

A composition of N-vinyl-pyrrolidone has a property to apt to polymerize during keeping, transportation, storage, and the like.

Therefore, the addition of ammonia, N,N'-sec-butyl-p-phenylenediamine, sodium hydroxide, and the like has been proposed aiming at suppression of polymerization (JP-A-8-506580).

In addition, there is such description that other amines with N,N'-sec-butyl-p-phenylenediamine may be used to prevent polymerization, however, there is no description or suggestion on what kind of amines are suitable for stability of color valency (APHA) (JP-A-7-252221).

### Disclosure of Invention

N,N'-sec-butyl-p-phenylenediamine (an aromatic amine having phenylenediamine skeleton) described in JP-A-8-506580 exhibits only low effect as a stabilizer when used in small amount, therefore the addition thereof in large quantity is required to express the effect. However, our study clarified that the addition of the compound in large quantity causes coloring of a product.

In addition, in JP-A-7-252221, N,N'-sec-butyl-p-phenylenediamine or the like was added to prevent polymerization in a formation step and not to maintain pH or reduce color valency (APHA) aiming at stability. Namely, it is not aimed at storage stability in the present invention, and thus it is hard to say that a problem of storage stability is solved.

Reference is made also to the following documents:
- D1:: US 5 461 159 A (HUCKESTEIN ET AL) 24 October 1995 (1995-10-24)
- D2:: EP 0 467 851 A (CIBA-GEIGY AG)22 January 1992 (1992-01-22)
- D3:: US 5 258 138 A (GATECHAIR ET AL) 2 November 1993 (1993-11-02)
- D4:: US 3 959 358 A (JURSICH ET AL) 25 May 1976 (1976-05-25)
- D5:: WO 00/37412 A (RHODIA CHIMIE; LARTIGUE-PEYROU, FRANCOISE) 29 June 2000 (2000-06-29)
- D6:: WO 99/55797 A (RHODIA CHIMIE ; LARTIGUE-PEYROU, FRANCOISE)4 November 1999 (1999-11-04)
- D7 :: WO 02/057372 A (ISP INVESTMENTS INC) 25 July 2002 (2002-07-25)
- D8:: EP 1 518 916 A (BASF AKTIENGESELLSCHAFT) 30 March 2005 (2005-03-30)
- D9:: DE 103 46 135 A1 (BASF AG) 7 October 2004 (2004-10-07)
- D10:: US-B1-6 649 717 (PREISS THOMAS ET AL) 18 November 2003 (2003-11-18)
- D11:: US 2005/079228 A1 (JAISWAL ASHISH ET AL) 14 April 2005 (2005-04-14)
- D12:: US-A-5 340 488 (ADAMS ET AL) 23 August 1994 (1994-08-23)

Document D1 discloses in column 3 lines 36-41 that tertiary amines are usually added to vinylpyrrolidone as stabilizer against premature polymerization.

Document D2 discloses certain amines (N-hydrocarbonyl substituted) as stabilizers of polymerizable monomers like N-vinylpyrrolidone (see page 10 lines 5-16 and claim 1).

Document D3 discloses also certain amines as stabilizers of vinyl monomers like N-vinylpyrrolidone (see claim 1 and column 11 lines 42-44).

Document D4 discloses the addition of amine-type compounds preventing peroxide initiated polymerization of acrylate esters (see claim 1 and column 2 lines 54-59).

Document D5 discloses composition for inhibiting radical polymerization of ethylenically unsaturated aliphatic monomers like N-vinylpyrrolidone comprising a diphenylamine derivative (see claim 1 and page 4 line 37).

Document D6 discloses composition for inhibiting radical polymerization of ethylenically unsaturated aliphatic monomers like N-vinylpyrrolidone comprising a bencenetriamine derivative (see claim 1 and page 4 line 38).

Document D7 discloses the stabilization of vinyl lactams with the use of aliphatic primary, secondary mono- or polyamines (see claim 1).

Document D8 discloses the use of radical inhibitors as stabilizators for compounds like N-vinylpyrrolidone (claim 1 and paragraph [0097]).

Document D9 discloses the use of radical inhibitors as stabilizators for compounds like N-vinylpyrrolidone (claim 1 and paragraphs [0101] and [0117] - [0119]).

Document D10 discloses the use of ammonium salts as stablizers of N-vinyl compounds like N-vinylpyrrolidone (page 2 line 51 and claim 1).

On the other hand, N-vinylpyrrolidones are frequently used as gelling agent in compositions, which may contain amines. This is the case of D11 where amines have been used as antioxidants (paragraphs [0035]-[0037]) or as suitable pH-modifying agents (par. [0040]) or of D12 where 1-vinylpyrrolidone is mixed with primary, secondary or tertiary amines (see claim 1 and examples).

N-vinyl-pyrrolidone has conventionally been well-known to be labile to radical polymerization, and thus paying close attention is required during keeping, transportation and storage. Accordingly, it is an object of the present invention to make possible stable storage of N-vinyl-pyrrolidone for a long period.

The present invention relates to a composition of N-vinyl-pyrrolidone, characterized by containing N-vinyl-pyrrolidone and at least one amine selected from the group consisting of alkanol amine, methylamine, ethylamine, isopropylamine, t-butylamine, dimethylamine, diethylamine, and cyclohexylamine, wherein content of the amine is in a range of not lower than 1 ppm and not higher than 3 % by weight, based on the N-vinyl-pyrrolidone, and content of N-vinyl-pyrrolidone is not lower than 80 % by weight in total composition.

According to the present invention, a composition of N-vinyl-pyrrolidone having small pH variation, and small increase in color valency (APHA) and superior storage stability for a long period, can be provided, by compounding specified amines and limiting pH range, if necessary.

### Best Mode for Carrying Out the Invention

We have intensively studied on stable storage of N-vinyl-pyrrolidone for a long period, and have found that variation of pH or color valency (APHA) can be suppressed and stable storage is possible for a long period at a temperature range of 30°C, by the addition of specified amines to N-vinyl-pyrrolidone, and adjusting pH thereof in a range of 8 to 12, if necessary. In this connection, use of "preservation" instead of "storage" is also within the scope of the present invention. The present invention also includes not only simple storage but also transfer or transportation.

Content of N-vinyl-pyrrolidone in a composition of the N-vinyl-pyrrolidone is not especially limited as long as being not lower than 80% by weight in total composition, however, preferably not lower than 85% by weight, more preferably not lower than 90% by weight and most preferably not lower than 95% by weight. The content within this range enables to sufficiently express storage stability of the composition.

A buffer agent or a deodorant, or water or alcohols as a solvent may be added into the present composition in addition to N-vinyl-pyrrolidone, within a range not to lower storage stability over a long period.

Amines to be added in the present invention as an amine compound (A) not having phenylenediamine skeleton, include alkanol amines such as monoethanol amine (MEA), diethanol amine, and triethanol amine; methylamine (MA), ethylamine (EA), isopropylamine, t-butylamine (tBA), dimethylamine, diethylamine, triethylamine, and cyclohexylamine. An organic amine compound is preferable in view of excellent stabilization effect, and furthermore, an organic amine compound having a vapor pressure at 20°C of not lower than 3×10⁻⁶ hPa is preferable. Specific examples thereof include aliphatic amines and alkanol amines.

Aromatic amines (B) which can be used in combination with the amine compound (A), in particular, aliphatic amines and alkanol amines, include any amines as long as having an aromatic ring, specifically aromatic amines such as aniline, toluidine, benzylamine, 2-naphthylamine, p-aminobenzoic acid, and N,N'-di-sec-butyl p-phenylenediamine (KEROBIT trade mark of BASF AG); heterocyclic aromatic amines such as 4-aminoquinoline; and the like are included.

The amine compound (A) may be used singly or as in a mixed form of two or more kinds. Furthermore, it may be used in combination with an aromatic amine (B), and in this case, two or more kinds of aromatic amines (B) may be added.

Content of the amine compound (A) based on the total composition depends on the kind of the amine to be added, and can be determined within a range enabling to efficiently express storage stability for a long period.

The addition amount of the amine compound (A) may be controlled in compounding so that pH determined by a specified pH measurement method, after storage at 30°C for 3 months under air atmosphere is in a range of 8 to 12, and preferably in a range of not lower than 1 ppm and not higher than 3% by weight is enough, and preferably in a range of not lower than 1 ppm and not higher than 1% by weight, more preferably in a range of not lower than 3 ppm and not higher than 5000 ppm, and most preferably in a range of not lower than 5 ppm and not higher than 1000 ppm, based on N-vinyl-pyrrolidone. By controlling the amount within this range, the effect of excellent storage stability of the composition for a long period can sufficiently be fulfilled.

On the other hand, ratio of an aromatic amine (B) to the amine compound (A) depends on a kind of an amine to be added, however, can be determined among the combinations with the amine compound (A) to afford to efficiently express storage stability. Specifically, the aromatic amine (B) is not higher than 1/2, preferably not higher than 1/5 and most preferably not higher than 1/10 of the amine compound (A), in weight ratio. It is because this range enables to express stabilization effect of the composition. The addition amount satisfying the above condition and in a range of, usually not lower than 1 ppm and not higher than 1% by weight is enough, and preferably it is in a range of not lower than 1 ppm and not higher than 5000 ppm, more preferably in a range of not lower than 3 ppm and not higher than 1000 ppm, and most preferably in a range of not lower than 3 ppm and not higher than 500 ppm, based on N-alkenyl carboxylic acid tertiary amides.

To make the composition furthermore stable, pH is controlled preferably in a range of 8 to 11, more preferably 8.5 to 10.5.

A composition of the present invention, in some cases, keeps stability at a high temperature of 90°C over 2 months, and thus can be said as a composition with very high stability.

Means to produce a composition of the present invention includes just the addition of specified amines to a composition of highly pure N-vinyl-pyrrolidone obtained via a purification step such as distillation, or to a composition of N-vinyl-pyrrolidone obtained without purification. The addition order of the amine compound (A) and the aromatic amine (B) is not especially limited, and the simultaneous addition is also possible.

For storage of a composition of N-vinyl-pyrrolidone, a sealed tank, drum, and the like can be used.

Atmosphere of the vapor phase part of a storage container of a composition of N-vinyl-pyrrolidone used in the present invention is not especially limited, however, handling by avoiding contact with acidic gas such as carbon dioxide gas is preferable, specifically, inert gas such as nitrogen gas is preferable. A composition of N-vinyl-pyrrolidone may be charged in a container after filling with inert gas, in advance, or inert gas may be filled after a composition of N-vinyl-pyrrolidone is charged.

### Examples

The present invention is explained more specifically by referring to Examples below.

An acceleration test method and a measurement method for pH and color valency (APHA) to confirm stability are shown below.

### (Test methods)

A composition sample of 100 g is charged in a 100 mL screw tube, which is capped and stored in an oven (produced from Sanyo Electric Co., Ltd.) set at 30°C under air atmosphere.

Property values are measured by the following methods, before the storage and after three months have passed.

### (Measurement method for pH)

Deionized water of 45 g which is adjusted to have a pH of 6 to 7 is weighed into a 50 mL screwed tube, in which 5 g of the sample is then weighed and mixed to produce a 10% by weight of an aqueous solution of the composition. A pH meter is set within 1 minute, and after stirring at 200 rpm, at 25°C for 1 minute, it is subjected to standing still and pH value at this point is read.

A pH meter (F-12 model; electrode type #6366-10D, produced from Horiba, Ltd.)

### (Measurement method for APHA color valency)

A color test reagent (Color 1000: produced from Wako Pure Chemical Industries, Ltd.) is diluted with deionized water to prepare various color standard solutions. A composition sample of 25 mL is charged in a 25 mL colorimetric tube, and APHA No. showing the same color, compared with standard solutions, is determined as measurement value.

### Examples 1 to 12 and Comparative Examples 1 to 4

NVP is added with various amines, and pH and color valency (APHA) thereof are measured at the start of storage and after 3 months have passed, whose results are shown in the following Table 1.

In this connection, NVP used in Examples 1 to 14 and Comparative Examples 1 to 6 are commercial NVP (containing 10 ppm of a stabilizer: N,N'-sec-butyl-p-phenylenediamine) after purification treatment such as distillation, and after removal of the stabilizer. As an example of such purification treatment, refer to Japanese patent No. 3435598.

**TABLE 1**

| | | Amines | | Initial value | | After 3 months | |
|---|---|---|---|---|---|---|---|
| | Composition | Amine comp. (A) | Aromatic amine comp.(B) | pH | Color valency | pH | Color valency |
| | (%) | (ppm) | (ppm) | | (APHA) | | (APHA) |
| Exp.1 | NVP (100) | MEA(100) | - | 10.3 | ≦5 | 9.8 | 35 |
| Exp.2 | ↑ | MA(500) | - | 10.7 | ≦5 | 10.0 | 35 |
| Exp.3 | ↑ | EA(500) | - | 10.3 | ≦5 | 9.7 | 35 |
| Exp.4 | ↑ | tBA(10) | - | 10.2 | <5 | 9.5 | 40 |
| Exp.5 | ↑ | MEA(50) | NN(10) | 10.3 | ≦5 | 9.6 | 40 |
| Exp.6 | ↑ | MA(300) | NN(10) | 10.5 | <5 | 10.0 | 30 |
| Exp.7 | ↑ | EA(1000) | NN(100) | 11.1 | ≦5 | 10.5 | 45 |
| Exp.8 | ↑ | tBA(100) | NN(10) | 10.4 | <5 | 9.9 | 35 |
| Exp.9 | NVP (90)/ Deionized water (10) | MEA(100) | - | 10.3 | ≦5 | 9.5 | 40 |
| Exp.10 | ↑ | MA(100) | - | 10.3 | <5 | 9.1 | 45 |
| Exp.11 | NVP (100) | MA(50) | - | 10.4 | ≦5 | 9.2 | 40 |
| | | MEA(50) | | | | | |
| Exp.12 | ↑ | MA(50) | NN(10) | 10.5 | ≦5 | 9.2 | 40 |
| | | MEA(50) | | | | | |
| Comp. Exp.1 | ↑ | - | - | 9.7 | ≦5 | 6.3 | 200 |
| Comp. Exp.2 | ↑ | - | NN(100) | 10.4 | <5 | 6.9 | 200 |
| Comp. Exp.3 | ↑ | - | NN(500) | 10.7 | ≦5 | 7.1 | 200 |
| Comp. Exp.4 | ↑ | - | NN (5 wt%) | 12.5 | 40 | 9.6 | 350 |

In this Table, "NN" represents N,N'-sec-butyl-p-phenylenediamine.

As is shown in Examples 1 to 12, by the addition of the amine compound (A) and the aromatic amine (B), if necessary, pH value little lowered compared with the value before starting the storage, and furthermore, variation range of color valency (APHA) could be controlled within 50, and therefore, these specified amines were found to significantly contribute to stability.

On the contrary, in Comparative Examples 1 to 3, pH decreased largely and variation range of color valency (APHA) was also large, due to no addition of the amine compound (A), and stability can be said insufficient.

Further, in Comparative Example 4, variation range of pH, in particular, color valency (APHA) was large, due to the excess addition of an amine having phenylenediamine skeleton, and stability was also insufficient.

From these results of the acceleration test, the present invention is assumed to be excellent even under a usual storage method.

### Example 13 and Comparative Example 5

NVP was charged, in the composition shown in Table 2, in a 200 kg content chemical drum (an inner bag was made of polyethylene). Values of pH and color valency (APHA) thereof were measured at the start of storage and after 3 months have passed, whose results are shown in Table 2. Storage condition was at room temperature (20°C to 25°C).

### Example 14 and Comparative Example 6

NVP was charged in the composition shown in Table 2, in an 18 L hybrid drum and nitrogen gas was then filled and the drum was sealed. Values of pH and color valency (APHA) thereof were measured at the start of storage and after 3 months have passed, whose results are shown in Table 2. Storage condition was at room temperature (20°C to 25°C).

**TABLE 2**

| | | Amines | | Initial value | | After 3 months | |
|---|---|---|---|---|---|---|---|
| | Composition | Amine comp. (A) | Aromatic amine comp.(B) | pH | Color valency | pH | Color valency |
| | (%) | (ppm) | (ppm) | | (APHA) | | (APHA) |
| Exp.13 | NVP(100) | MEA(50) | NN(10) | 10.7 | 10 | 10.5 | 50 |
| Exp.14 | NVP(100) | MEA(50) | NN(10) | 10.7 | 10 | 10.8 | 45 |
| Comp. Exp. 5 | NVP(100) | - | - | 8.5 | 5 | 6.4 | 300 |
| Comp. Exp.6 | NVP(100) | - | - | 10.8 | 10 | 7.0 | 300 |

As is shown in Table 2, a composition of the present invention was confirmed to be excellent even when stored under practical storage condition.

The above contents are preferable embodiments of the present invention.

## Claims

1. A composition of N-vinyl-pyrrolidone, comprising at least one amine selected from the group consisting of an alkanol amine, methylamine, ethylamine, isopropylamine, t-butylamine, dimethylamine, diethylamine, and cyclohexylamine, wherein content of the amine is in a range of not lower than 1 ppm and not higher than 3% by weight, based on weight of the N-vinyl-pyrrolidone, and content of N-vinyl-pyrrolidone is not lower than 80% by weight in total composition.

2. The composition of claim 1, wherein the amine is an alkanol amine.

3. The composition of claim 2, wherein the alkanol amine is an amine having a vapor pressure at 20°C of not lower than 3.10⁻⁶ hPa.

4. The composition of claim 2, wherein the alkanol amine is at least one member selected from a group consisting of monoethanol amine, diethanol amine, and triethanol amine.

5. The composition of claim 1, further comprising an aromatic amine (B).

6. The composition of claim 5, wherein the aromatic amine (B) is at least one member selected from the group consisting of aniline, toluidine, benzylamine, 2-naphthylamine, N,N'-di-sec-butyl p-phenylenediamine and p-aminobenzoic acid.

7. The composition of claim 1, further comprising a solvent.

8. The composition of claim 7, wherein content of the solvent is up to 20% by weight based on total weight of the resultant composition.

9. The composition of claim 1, wherein pH determined by a specified pH measurement method, after subjecting the composition to an acceleration test at 30°C for 3 months under air atmosphere, is in a range of 8 to 12.

10. The composition of claim 1, wherein variation of APHA color valency determined by a specified APHA color valency measurement method, after subjecting the composition to an acceleration test at 30°C for 3 months under air atmosphere, is within 50 compared with the value before storage.

11. Use of at least one amine selected from the group consisting of an alkanol amine, methylamine, ethylamine, isopropylamine, t-butylamine, dimethylamine, diethylamine, and cyclohexylamine, to prevent polymerisation of N-vinyl-pyrrolidone, wherein the amine is used in a range of not lower than 1 ppm and not higher than 3% by weight, based on weight of the N-vinyl-pyrrolidone.

## Patentansprüche

1. Eine Zusammensetzung des N-Vinyl-Pyrrolidons, aufweisend wenigstens ein Amin, ausgewählt aus der Gruppe bestehend aus Alkanolamin, Methylamin, Ethylamin, Isopropylamin, t-Butylamin, Dimethylamin, Diethylamin und Cyclohexylamin, wobei der Gehalt des Amins in einem Bereich von nicht weniger als 1 ppm und nicht größer als 3 Gew.-% liegt, bezogen auf das Gewicht des N-Vinyl-Pyrrolidons, und der Gehalt des N-Vinyl-Pyrrolidons nicht geringer als 80 Gew.-% der Gesamtzusammensetzung ist.

2. Zusammensetzung nach Anspruch 1, wobei das Amin ein Alkanolamin ist.

3. Zusammensetzung nach Anspruch 2, wobei das Alkanolamin ein Amin ist, welches bei 20°C einen Dampfdruck von nicht weniger als 3.10⁻⁶ hPa hat.

4. Zusammensetzung nach Anspruch 2, wobei das Alkanolamin wenigstens ein Mitglied ist ausgewählt aus der Gruppe bestehend aus Monoethanolamin, Diethanolamin und Triethanolamin.

5. Zusammensetzung nach Anspruch 1, weiterhin aufweisend ein aromatisches Amin (B). ,

6. Zusammensetzung nach Anspruch 5, wobei das aromatische Amin (B) wenigstens ein Mitglied ist, ausgewählt aus der Gruppe bestehend aus Anilin, Toluidin, Benzylamin, 2-Naphthylamin, N,N'-Di-sec-butyl-p-phenylendiamin und p-Aminobenzoesäure.

7. Zusammensetzung nach Anspruch 1, weiterhin aufweisend ein Lösungsmittel.

8. Zusammensetzung nach Anspruch 7, wobei der Gehalt des Lösungsmittels bis zu 20 Gew.-% ist, bezogen auf das Gesamtgewicht der resultierenden Zusammensetzung.

9. Zusammensetzung nach Anspruch 1, wobei der pH, bestimmt durch eine angegebene pH-Messmethode im Bereich von 8 bis 12 ist, nachdem man die Zusammensetzung einem Beschleunigungstest bei 30°C für 3 Monate unter Luftatmosphäre ausgesetzt hat.

10. Zusammensetzung nach Anspruch 1, wobei die Abweichung des APHA Farbwertes, bestimmt nach einem angegebenen APHA Farbwertmessverfahren innerhalb von 50 liegt verglichen mit dem Wert vor der Lagerung, nachdem man die Zusammensetzung einem Beschleunigungstest bei 30°C für 3 Monate unter Luftatmosphäre ausgesetzt hat.

11. Verwendung wenigstens eines Amins, ausgewählt aus der Gruppe bestehend aus Alkanolamin, Methylamin, Ethylamin, Isopropylamin, t-Butylamin, Dimethylamin, Diethylamin und Cyclohexylamin zur Verhinderung der Polymerisation von N-Vinylpyrrolidon, wobei das Amin im Bereich von nicht weniger als 1 ppm und nicht mehr als 3 Gew.-% verwendet wird, bezogen auf das Gewicht des N-Vinylpyrrolidons.

## Revendications

1. Composition de N-vinyl-pyrrolidone, comprenant au moins une amine sélectionnée dans le groupe constitué d'une alcanol amine, de la méthylamine, de l'éthylamine, de l'isopropylamine, de la t-butylamine, de la diméthylamine, de la diéthylamine, et de la cyclohexylamine, dans laquelle la teneur en amine se trouve dans une plage qui n'est pas inférieure à 1 ppm et pas supérieure à 3 % en poids, sur la base du poids de la N-vinyl-pyrrolidone, et la teneur en N-vinyl-pyrrolidone n'est pas inférieure à 80 % en poids dans la composition totale.

2. Composition selon la revendication 1, dans laquelle l'amine est une alcanol amine.

3. Composition selon la revendication 2, dans laquelle l'alcanol amine est une amine ayant une pression de vapeur à 20 °C qui n'est pas inférieure à 3.10⁻⁶ hPa.

4. Composition selon la revendication 2, dans laquelle l'alcanol amine est au moins un membre sélectionné dans un groupe constitué de la monoéthanol amine, de la diéthanol amine, et de la triéthanol amine.

5. Composition selon la revendication 1, comprenant en outre une amine aromatique (B).

6. Composition selon la revendication 5, dans laquelle l'amine aromatique (B) est au moins un membre sélectionné dans le groupe constitué de l'aniline, de la toluidine, de la benzylamine, de la 2-naphtylamine, de la N,N'-di-sec-butyl p-phénylènediamine et de l'acide p-aminobenzoïque.

7. Composition selon la revendication 1, comprenant en outre un solvant.

8. Composition selon la revendication 7, dans laquelle la teneur en solvant représente jusqu'à 20 % en poids sur la base du poids total de la composition obtenue.

9. Composition selon la revendication 1, dans laquelle le pH déterminé par un procédé de mesure du pH spécifié, après la soumission de la composition à un test de vieillissement accéléré à 30 °C pendant 3 mois sous atmosphère d'air, se trouve dans une plage de 8 à 12.

10. Composition selon la revendication 1, dans laquelle la variation de la valence chromatique APHA déterminée par un procédé de mesure de la valence chromatique APHA spécifié, après la soumission de la composition à un test de vieillissement accéléré à 30 °C pendant 3 mois sous atmosphère d'air, est dans un intervalle de 50 par rapport à la valeur avant le stockage.

11. Utilisation d'au moins une amine sélectionnée dans le groupe constitué d'une alcanol amine, de la méthylamine, de l'éthylamine, de l'isopropylamine, de la t-butylamine, de la diméthylamine, de la diéthylamine, et de la cyclohexylamine, pour empêcher la polymérisation de la N-vinyl-pyrrolidone, dans laquelle l'amine est utilisée dans une plage qui n'est pas inférieure à 1 ppm et pas supérieure à 3 % en poids, sur la base du poids de la N-vinyl-pyrrolidone.
